# EUROPEAN PATENT APPLICATION

(11) **EP 2 218 783 A1**
(43) Date of publication of application: **18.08.2010**
(21) Application number: 09001621.3
(22) Date of filing: 05.02.2009
(51) Int. Cl.: C12N 15/57, C07K 14/33, C12N 9/52, C12N 5/10, C12N 15/63, A61K 38/01, A61K 38/16, C12P 21/00, G01N 33/68

(54) **Novel method for the manufacturing of neurotoxins**

(71) Applicant: Merz Pharma GmbH & Co. KGaA, 60318 Frankfurt (DE)
(72) Inventor: Wilk, Thomas, Dr., 67117 Limburgerhof (DE)
(74) Representative: Ricker, Mathias

(57) **Abstract**

Novel method for the producing Clostridial neurotoxins, comprising the step of expressing nucleic acid sequence encoding the neurotoxin and comprising a cis-acting hydrolase element, novel Clostridial neurotoxins and nucleic acid sequences encoding such Clostridial neurotoxins.

## Description

### FIELD OF THE INVENTION

This invention relates to a novel method for producing Clostridial neurotoxins. The method comprises the step of expressing a nucleic acid sequence encoding the neurotoxin and comprising a cis-acting hydrolase element. The invention further relates to novel Clostridial neurotoxins and nucleic acid sequences encoding such Clostridial neurotoxins.

### BACKGROUND OF THE INVENTION

Clostridium is a genus of Gram-positive bacteria that includes important pathogens, such as *Clostridium botulinum* and *Clostridium tetani,* which produce neurotoxins such as botulinum toxin and tetanus toxin, respectively, which are among the strongest toxins known to man.

Both botulinum toxin and tetanus toxin act at several sites within the central nervous system. Botulinum toxin blocks the release of the neurotransmitter acetylcholine from axons at a neuromuscular junction, while tetanus toxin blocks inhibitory impulses by interfering with the release of neurotransmitters at the presynaptic junctions of inhibitory motor nerve endings.

In *Clostridium botulinum,* the botulinum toxin is formed as a protein complex comprising the neurotoxic component and non-toxic proteins, and complexes with 450 kDa and the 900 kDa are obtainable from cultures of *Clostridium botulinum.*

The neurotoxic components of botulinum toxin is a two-chain polypeptide with a molecular weight of approximately 150 kDa, with a 100 kDa heavy chain joined by a disulfide bond to a 50 kDa light chain. The heavy chain is responsible for entry into the neuronal cell, while the light chain comprises an endopeptidase activity responsible for cleaving one or more proteins that is/are part of the so-called SNARE-complex involved in the process resulting in the release of neurotransmitter into the synaptic cleft.

In recent years, Botulinum neurotoxins have become the standard agents in the treatment of focal dystonias and spastic indications. Preparations comprising botulinum toxin complexes are commercially available, e.g. from Ipsen Ltd. (Dysport®) or Allergan Inc. (Botox®). A high purity neurotoxic component, free of any other botulinum protein, is e.g. available from Merz Pharmaceuticals GmbH, Frankfurt (Xeomin®).

Treatment of patients generally involves injection of the neurotoxin, or neurotoxic component, into affected muscle tissue, bringing the agent near the neuromuscular end plate, i.e. close to the cellular receptor mediating its uptake into the nerve cell controlling said affected muscle. Various degrees of neurotoxin spread have been observed. This spread is thought to correlate with the injected amounts and the particular preparation of neurotoxin injected. Resulting from the spread, systematic side effects caused by the inhibition of acetylcholine release, may be observed at nearby muscle tissue. The incidents of unintended paralysis of untreated muscles can largely be avoided by reducing the injected doses to the therapeutically relevant level. Overdosing may also be a problem with regard to the patients' immune system, as the injected neurotoxin may trigger the formation of neutralizing antibodies. If this occurs, the neurotoxin will be inactivated without being able to relieve the involuntary muscle activity.

Differences in the dose equivalents of preparations such as available sales products or batches produced during the manufacturing process, commonly a fermentation process, pose an increased risk for patients through possible side effects and the development of immunity. Therefore, it is of crucial importance to determine the biological activity of a Clostridial neurotoxin contained in said sales products or production batches reliably (i.e. without significant variation) and as accurately as possible, in order to adjust the neurotoxin concentration to a reliable effective dose for the benefit of the patient. This may also serve as an incentive to the manufacturers to offer formulations allowing optimum exploitation of biological activity for different therapeutic purposes.

Predominantly, Clostridial neurotoxins are produced by fermentation processes using appropriate Clostridium strains. Methods for the fermentation process using *Clostridium botulinum* strains are well known in the art (see, for example, Siegel and Metzger, Appl. Environ. Microbiol. 38 (1979) 606-611; Siegel and Metzger, Appl. Environ. Microbiol. 40 (1980) 1023-1026).

Additionally, Clostridial neurotoxins can be produced in heterologous cells, i.e. be produced recombinantly by expressing nucleic acid sequences encoding a neurotoxin in an appropriate host cells. Methods for the recombinant expression of Clostridial neurotoxin in E. coli are well known in the art (see, for example, WO 00/12728, WO 01/14570, or WO 2006/076902). In certain cases, the light and heavy chains are separately obtained, and then reconstituted in vitro (see WO 95/32738).

Furthermore, Clostridial neurotoxins have been expressed in eukaryotic expression systems, such as in *Pichia pastoris, Pichia methanolica, S. cerevisiae*, insect cells and mammalian cells (see WO 2006/017749).

In all these expression systems, a proteolytic cleavage of the single chain neurotoxin is required, either by host cell enzymes during fermentation, or by adding proteolytic enzymes to the raw protein material isolated after fermentation, in order to generate the final biologically active neurotoxin protein comprising a light chain and a heavy chain linked by a disulfide bond.

### OBJECTS OF THE INVENTION

It was an object of the invention to improve the methods of the prior art and to develop a reliable and accurate method for manufacturing and obtaining novel Clostridial neurotoxins with improved properties without the need of a proteolytic cleavage of the single-chain neurotoxin. Such a method and novel Clostridial neurotoxins would also serve to satisfy the great need for a safe and effective administration.

### SUMMARY OF THE INVENTION

Surprisingly it has been found that biologically active Clostridial neurotoxins can be obtained recombinantly in functional form from eukaryotic host cells by expressing a nucleic acid construct comprising nucleic acid sequences encoding the Clostridial neurotoxin light and heavy chains connected by a nucleic acid sequence encoding a cis-acting hydrolase element.

Thus, in one aspect, the present invention relates to a recombinant Clostridial neurotoxin comprising: (i) a functionally active Clostridial neurotoxin light chain; and (ii) a functionally active Clostridial neurotoxin heavy chain, **characterized in that** said light chain comprises a cleaved cis-acting hydrolase element at the C-terminus of said light chain, and said heavy chain comprises a proline residue located at the N-terminus of said heavy chain.

In other aspects, the present invention relates to a nucleic acid sequence encoding the recombinant Clostridial neurotoxin of the present invention, a method of obtaining a nucleic acid sequence of the present invention, vectors, host cells and methods for producing the recombinant Clostridial neurotoxin of the present invention, and pharmaceutical compositions.

### DETAILED DESCRIPTION OF THE INVENTION

The present invention may be understood more readily by reference to the following detailed description of the invention and the examples included therein.

In the context of the present invention, the term "Clostridial neurotoxin" refers to a natural neurotoxin obtainable from bacteria of the class Clostridia, including *Clostridium tetani* and *Clostridium botulinum,* or to a neurotoxin obtainable from alternative sources, including from recombinant technologies or from genetic or chemical modification. Particularly, the Clostridial neurotoxins have endopeptidase activity.

In the context of the present invention, the term "recombinant Clostridial neurotoxin" refers to a composition comprising a Clostridial neurotoxin, that is obtained by expression of the neurotoxin in a heterologous cell such as *P. pistoris,* and including, but not limited to, the raw material obtained from a fermentation process (supernatant, composition after cell lysis), a fraction comprising a Clostridial neurotoxin obtained from separating the ingredients of such a raw material in a purification process, an isolated and essentially pure Clostridial neurotoxin, and a formulation for pharmaceutical and/or aesthetic use comprising a Clostridial neurotoxin and additionally pharmaceutically acceptable solvents and/or excipients.

In the context of the present invention, the term "functionally active" refers to the property of a recombinant Clostridial neurotoxin to perform the biological functions of a naturally occurring Clostridium botulinum neurotoxin to at least about 50%, particularly to at least about 60%, to at least about 70%, to at least about 80%, and most particularly to at least about 90%, where the biological functions include, but are not limited to, association of light and heavy chain to form the two-chain neurotoxin protein, entry of the neurotoxin into a neuronal cell, release of the light chain from the two-chain neurotoxin, and endopeptidase activity of the light chain. Methods for determining a neurotoxic activity can be found, for example, in WO 95/32738, which describes the reconstitution of separately obtained light and heavy chains of tetanus toxin and botulinum toxin.

In the context of the present invention, the term "Clostridial neurotoxin light chain" refers to that part of a Clostridial neurotoxin that comprises an endopeptidase activity responsible for cleaving one or more proteins that is/are part of the so-called SNARE-complex involved in the process resulting in the release of neurotransmitter into the synaptic cleft: In naturally occurring Clostridial neurotoxins, the light chain has a molecular weight of approx. 50 kDa.

In the context of the present invention, the term "Clostridial neurotoxin heavy chain" refers to refers to that part of a Clostridial neurotoxin that is responsible for entry of the neurotoxin into the neuronal cell: In naturally occurring Clostridial neurotoxins, the heavy chain has a molecular weight of approx. 100 kDa.

In particular embodiments, the light chain and the heavy chain of the present invention are linked by a disulfide bond.

In the context of the present invention, the term "cis-acting hydrolase element", or "intraribosomal cleavage sequence" refers to oligopeptide moieties that, when present in a parental contiguous single-chain sequence between two proteins, cause the co-translational self-processing of the contiguous single-chain such that each constituent protein is generated as a discrete translation product (see: de Felipe et al., Trends Biotechnol. 24 (2006) 68-75). Such cis-acting hydrolase elements are found, for example, in certain viral genomes, such as the 2A sequence in the genome of the foot-and-mouth disease virus (FMDV), or *Trypansoma spp..* The 2A and 2A-like sequences, or cis-acting hydrolase elements in general, consist of between about 16 and 25 amino acid residues with a consensus sequence at the C-terminal end consisting of "DxEyNPG*P", with x being predominantly taken from the list of I, L and V, and with y being predominantly taken from the list of E, K, L, M, Q, S and T, and with the self-processing cleavage occurring between G and P (see *).

In the context of the present invention, the term "cleaved cis-acting hydrolase element" refers to that certain part of a cis-acting hydrolase element that remains at the C-terminus of the N-terminal protein part of a parental contiguous single-chain sequence after expression. On the other side, the C-terminal part of the parental contiguous single-chain sequence will carry a proline residue. In other words, on expression of a nucleic acid sequence encoding a parental contiguous single-chain sequence that can schematically be defined as "domain1-(cis-acting hydrolase element)-domain2" in a eukaryotic host cell, two protein fragments are obtained that can schematically be defined as "domain1-(cleaved cis-acting hydrolase element)" and "proline-domain2".

In one embodiment of the present invention, the recombinant Clostridial neurotoxin is a functional homologue of a *Clostridium botulinum* neurotoxin taken from the list of: *Clostridium botulinum* neurotoxin serotype A, B, C, D, E, F, and G.

In the context of the present invention, the term "*Clostridium botulinum* neurotoxin serotype A, B, C, D, E, F, and G" refers to neurotoxins obtainable from *Clostridium botulinum.* Currently, seven serologically distinct types, designated serotypes A, B, C, D, E, F and G, are known, including certain subtypes (e.g. A1, A2, and A3).

In a particular embodiment, the recombinant Clostridial neurotoxin is a functional homologue of a *Clostridium botulinum* neurotoxin serotype A.

In the context of the present invention, the term "functional homologue of a *Clostridium botulinum* neurotoxin" refers to a neurotoxin that differs in the amino acid sequence, or the nucleic acid sequence encoding the amino acid sequence, from a *Clostridium botulinum* neurotoxin but is still functionally active. On the protein level, a functional homologue will maintain key features of the corresponding *Clostridium botulinum* neurotoxin, such as key residues for the endopeptidase activity in the light chain, or for the attachment to the neurotoxin receptors in the heavy chain, but may contain one or more mutations comprising a deletion of one or more amino acids of the parental *Clostridium botulinum* neurotoxin, an addition of one or more amino acids to the parental *Clostridium botulinum* neurotoxin and/or a substitution of one or more amino acids of the parental *Clostridium botulinum* neurotoxin. Preferably, said deleted or added amino acids are consecutive amino acids. According to the teaching of the present invention, any number of amino acids may be added or deleted, as long as the neurotoxin is biologically active. For example, 1, 2, 3, 4, 5, up to 10, up to 15, up to 25, up to 50, up to 100, up to 200, up to 400, up to 500 amino acids or even more amino acids may be added or deleted. In certain aspects, the present invention refers to neurotoxins, with an addition of more than 500 amino acids, such as for example up to 600 or up to 800 additional amino acids, or even more additional amino acids. Accordingly, a derivative of the neurotoxin may be a biologically active fragment of a naturally occurring neurotoxin. This neurotoxin fragment may contain an N-terminal, C-terminal and/or one or more internal deletion(s). "Biologically active", as used in this context, means, said derivative is taken up into the nerve cell and is capable of denervating said nerve from the muscle or gland, to which it is connected. Methods for designing and constructing homologues of a *Clostridium botulinum* neurotoxin and for testing of such homologues for functionality are well known to anyone of ordinary skill in the art.

In particular embodiments, the functional homologue has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95%. Methods and algorithms for determining sequence identity and/or homology, including the comparison of variants having additions and/or insertions relative to a parental sequence, are well known to the practitioner of ordinary skill in the art. On the DNA level, the nucleic acid sequences encoding the functional homologue and the parental Clostridium neurotoxin may differ to a larger extent due to the degeneracy of the genetic code. It is known that the usage of codons is different between prokaryotic and eukaryotic organisms (see, for example, Table 1 in WO 2006/017749). Thus, when expressing a prokaryotic protein such as a Clostridium neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In particular embodiments, the functional homologue of the Clostridium neurotoxin is a derivative of the Clostridium neurotoxin.

In the context of the present invention, the term "derivative" and the term "variant" or "synthetic analogue" each individually refer to a neurotoxin that is a chemically, enzymatically or genetically modified derivative of a parental Clostridium neurotoxin, including chemically or genetically modified neurotoxin from *C*. *botulinum,* particularly of *C*. *botulinum* neurotoxin serotype A. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids, including modification occurring in the eukaryotic host cell used for expressing the derivate. An enzymatically modified derivative is one that is modified by the activity of enzymes, such as endo- or exoproteolytic enzymes, including by modification by enzymes of the eukaryotic host cell used for expressing the derivate. As pointed out above, a genetically modified derivative is one that has been modified by deletion or substitution of one or more amino acids contained in, or by addition of one or more amino acids (including polypeptides comprising between 2 and about 100 amino acids) to, the amino acid sequence of said Clostridium neurotoxin. Methods for making such chemically or genetically modified derivatives, and methods for identifying whether such derivatives maintain neurotoxic activity, are well known to anyone of ordinary skill in the art.

In one embodiment, the light chain of the recombinant Clostridial neurotoxin of the present invention consists of (a) amino acid residues 2 to 444 of *Clostridium botulinum* neurotoxin serotype A; or of (b) a functionally active variant of the light chain defined in (a), fused to a cleaved cis-acting hydrolase element at the C-terminus of said light chain (see Dasgupta & Dekleva, Biochimie 72 (1990) 661-664).

In another embodiment, the light chain of the recombinant Clostridial neurotoxin of the present invention consists of (a) amino acid residues 2 to 438 of *Clostridium botulinum* neurotoxin serotype A; or of (b) a functionally active variant of the light chain defined in (a), fused to a cleaved cis-acting hydrolase element at the C-terminus of said light chain (see Lacy & Stevens, J. Mol. Biol. 291 (1999) 1091).

In certain such embodiments, the light chain of the recombinant Clostridial neurotoxin of the present invention comprises a cleaved cis-acting hydrolase element at the C-terminus having the consensus formula:

(z)ₙ DxEyNPG

with z being independently any amino acid;
n being an integer between about 8 and about 17;
x being an amino acid taken from the list of I, L and V;
and with y being an amino acid taken from the list of E, K, L, M, Q, S and T.

In another embodiment, the heavy chain of the recombinant Clostridial neurotoxin of the present invention consists of an N-terminal proline linked to (a) amino acid residues 449 to 1296 of *Clostridium botulinum* neurotoxin serotype A; or of (b) a functionally active variant of the heavy chain defined in (a) (see Dasgupta & Dekleva, Biochimie 72 (1990) 661-664; Lacy & Stevens, J. Mol. Biol. 291 (1999) 1091).

In a particular embodiment of the present invention, the light chain has the amino acid sequence as found in SEQ ID NO: 1.

In a particular embodiment of the present invention, the heavy chain has the amino acid sequence as found in SEQ ID NO: 2.

In a further particular embodiment of the present invention, the light chain has the amino acid sequence as found in SEQ ID NO: 1, and the heavy chain has the amino acid sequence as found in SEQ ID NO: 2.

In another embodiment of the present invention, the recombinant Clostridial neurotoxin is a functional homologue of a *Clostridium tetani* neurotoxin.

In the context of the present invention, the term "functional homologue of a *Clostridium tetani* neurotoxin" refers to a neurotoxin that differs in the amino acid sequence, or the nucleic acid sequence encoding the amino acid sequence, from a *Clostridium tetani* neurotoxin but is still functionally active. On the protein level, a functional homologue will maintain key features of the corresponding *Clostridium tetani* neurotoxin, such as key residues for the endopeptidase activity in the light chain, or for the attachment to the neurotoxin receptors in the heavy chain, but may contain one or more mutations comprising a deletion of one or more amino acids of the parental *Clostridium tetani* neurotoxin, an addition of one or more amino acids to the parental *Clostridium tetani* neurotoxin and/or a substitution of one or more amino acids of the parental *Clostridium tetani* neurotoxin. Preferably, said deleted or added amino acids are consecutive amino acids. According to the teaching of the present invention, any number of amino acids may be added or deleted, as long as the neurotoxin is biologically active. For example, 1, 2, 3, 4, 5, up to 10, up to 15, up to 25, up to 50, up to 100, up to 200, up to 400, up to 500 amino acids or even more amino acids may be added or deleted. In certain aspects, the present invention refers to neurotoxins, with an addition of more than 500 amino acids, such as for example up to 600 or up to 800 additional amino acids, or even more additional amino acids. Accordingly, a derivative of the neurotoxin may be a biologically active fragment of a naturally occurring neurotoxin. This neurotoxin fragment may contain an N-terminal, C-terminal and/or one or more internal deletion(s). "Biologically active", as used in this context, means, said derivative is taken up into the nerve cell and is capable of denervating said nerve from the muscle or gland, to which it is connected. Methods for designing and constructing homologues of a *Clostridium tetani* neurotoxin and for testing of such homologues for functionality are well known to anyone of ordinary skill in the art.

In particular embodiments, the functional homologue has a sequence identity of at least about 40%, at least about 50%, at least about 60%, at least about 70% or most particularly at least about 80%, and a sequence homology of at least about 60%, at least about 70%, at least about 80%, at least about 90%, or most particularly at least about 95%. Methods and algorithms for determining sequence identity and/or homology, including the comparison of variants having additions and/or insertions relative to a parental sequence, are well known to the practitioner of ordinary skill in the art. On the DNA level, the nucleic acid sequences encoding the functional homologue and the parental Clostridium neurotoxin may differ to a larger extent due to the degeneracy of the genetic code. It is known that the usage of codons is different between prokaryotic and eukaryotic organisms (see, for example, Table 1 in WO 2006/017749). Thus, when expressing a prokaryotic protein such as a Clostridium neurotoxin, in a eukaryotic expression system, it may be necessary, or at least helpful, to adapt the nucleic acid sequence to the codon usage of the expression host cell, meaning that sequence identity or homology may be rather low on the nucleic acid level.

In one embodiment, the light chain of the recombinant Clostridial neurotoxin of the present invention consists of (a) amino acid residues 2 to 445, 446, 448, 449 or 454 of Clostridium tetani neurotoxin; or of (b) a functionally active variant of the light chain defined in (a), fused to a cleaved cis-acting hydrolase element at the C-terminus of said light chain (see Krieglstein et al., Eur. J. Biochem. 202 (1991) 41-51).

In another embodiment, the heavy chain of the recombinant Clostridial neurotoxin of the present invention consists of an N-terminal proline linked to (a) amino acid residues 450, 453, 455, 457, 460, or 461 to 1314 of Clostridium tetani neurotoxin; or of (b) a functionally active variant of the heavy chain defined in (a) (see Krieglstein et al., Eur. J. Biochem. 202 (1991) 41-51).

In a particular embodiment of the present invention, the light chain has the sequence as found in SEQ ID NO: 3, and the heavy chain has the sequence as found in SEQ ID NO: 4.

In certain embodiments of the present invention, the cleaved cis-acting hydrolase element is comprised in a cis-acting hydrolase element from (i) a virus taken from the list of: EMC-B, EMC-D, EMC-PV21, TME-GD7, TME-DA, DME-BEAN, Theiler's-Like Virus, Ljungan virus (174F), Ljungan virus (145SL), Ljungan virus (87-012), Ljungan virus (M1146), FMD-A10, FMD-A12, FMD-C1, FMD-O1G FMD-O1K, FMD-O (Taiwan), FMD-O/SK, FMD-SAT3, FMD-SAT2, ERAV, ERBV, ERV-3, PTV-1, PTV-2, PTV-3, PTV-4, PTV-5, PTV-6, PTV-7, PTV-8, PTV-9, PTV-10, PTV-11, CrPV, DCV, ABPV, ABPV isolate Poland 1, ABPV isolate Hungary 1, IFV, TaV, EEV, KBV, PnPV (a), PnPV (b), Ectropis oblique picorna-like virus (a), Ectropis oblique picorna-like virus (b), Providence virus (a), Providence virus (b), Providence virus (c), Bovine Rotavirus, Porcine Rotavirus, Human Rotavirus, Bombyx mori, Lymantria dispar, Dendrolimus punctatus; (ii) a Trypansoma spp. Repeated Sequence taken from the list of: T. brucei TSR1, T. brucei (CAB95325.1), T. brucei (CAB95342.1), T. brucei (CAB95559.1), T. cruzi AP Endonuclease, or (iii) a cis-acting hydrolase element that is a functional variant or a cis-acting hydrolase element listed in (i) or (ii).

In the context of the present invention, a cis-acting hydrolase element is a functional variant of another cis-acting hydrolase element (the reference cis-acting hydrolase element), when it causes the co-translational self-processing of a contiguous single-chain it is part of, such that each constituent protein of the contiguous single-chain is generated as a discrete translation product in essentially the same way as when using the reference cis-acting hydrolase element. Methods for designing and constructing variants of cis-acting hydrolase elements and for testing of such variants for functionality, e.g. by using the methods disclosed in de Felipe et al. (loc. cit.), are well known to anyone of ordinary skill in the art.

In certain embodiments, such functional variant is derived, either physically or theoretically, from such reference cis-acting hydrolase element by containing one or more mutations comprising a deletion of one or more amino acids of the reference cis-acting hydrolase element, an addition of one or more amino acids to the reference cis-acting hydrolase element and/or a substitution of one or more amino acids of the reference cis-acting hydrolase element.

In another aspect, the present invention relates to a nucleic acid sequence encoding the recombinant Clostridial neurotoxin of the present invention, comprising
(i) a nucleic acid sequence encoding a functionally active Clostridial neurotoxin light chain, (ii) a nucleic acid sequence encoding a cis-acting hydrolase element, and (iii) a nucleic acid sequence encoding a functionally active Clostridial neurotoxin heavy chain.

In particular embodiments, the nucleic acid sequences (i), (ii) and (iii) encode a single contiguous amino acid sequence.

In the context of the present invention, the term "single contiguous amino acid sequence" refers to an amino acid sequence that would, when expressed from the corresponding nucleic acid sequence in an appropriate host cell in the absence of a cis-acting hydrolase element activity, be formed as a single chain protein.

In particular embodiments, the single contiguous amino acid sequence comprises from N- to C-terminus the functionally active Clostridial neurotoxin light chain, the cis-acting hydrolase element, and the functionally active Clostridial neurotoxin heavy chain, and such format of the gene arrangement has been used throughout the disclosure and the Examples.

In other embodiments, the single contiguous amino acid sequence comprises from N- to C-terminus the functionally active Clostridial neurotoxin heavy chain, the cis-acting hydrolase element, and the functionally active Clostridial neurotoxin light chain. In such embodiments, the heavy chain will comprise, after self-processing, the cleaved cis-acting hydrolase element, and the light chain will comprise an N-terminal proline residue, either as an additional proline residue, or by using the proline residue occurring at position 2 of Botulinum toxin or Tetanus toxin, i.e. the N-terminal end of the processed light chain, in the construction of the cis-acting hydrolase element.

In certain embodiments, the nucleic acid sequence encoding the recombinant Clostridial neurotoxin of the present invention, comprises (i) a nucleic acid sequence encoding a functionally active Clostridial neurotoxin light chain, (ii) a nucleic acid sequence encoding a cis-acting hydrolase element having the consensus formula:

(z)ₙ DxEyNPGP

with z being independently any amino acid;
n being an integer between about 8 and about 17;
x being an amino acid taken from the list of I, L and V;
and with y being an amino acid taken from the list of E, K, L, M, Q, S and T.
   and (iii) a nucleic acid sequence encoding a functionally active Clostridial neurotoxin heavy chain.

In particular such embodiments, n is an integer between 8 and 17.

In a particular embodiment, the nucleic acid has the sequence as found in SEQ ID NO: 5.

In another aspect, the present invention relates to a method for obtaining the nucleic sequence of the present invention, comprising the step of inserting of a nucleic acid sequence encoding a cis-acting hydrolase element into a nucleic acid sequence encoding a Clostridial neurotoxin.

In a particular embodiment, the insertion is achieved by inserting the cis-acting hydrolase element into the nucleic acid sequence encoding wild-type Clostridial neurotoxin. In another particular embodiment, the insertion results in the deletion and/or modification of one or more amino acid residues of said wild-type Clostridial neurotoxin.

Another aspect of the present invention relates to a vector comprising a nucleic acid sequence of the present invention.

A further aspect of the present invention relates to a eukaryotic host cell comprising a nucleic acid sequence or a vector of the present invention.

Yet another aspect of the invention relates to a method for producing the recombinant Clostridial neurotoxin of the present invention, comprising the step of expressing a nucleic acid or vector of the present invention in a eukaryotic host cell, or cultivating a eukaryotic host cell of the present invention under conditions that result in the expression of the nucleic acid sequence.

In an additional aspect, the present invention relates to a method for detecting, isolating or purifying the recombinant Clostridial neurotoxin of the present invention, comprising the step of using an antibody with specificity for a cleaved cis-acting hydrolase element sequence.

Methods of generating and characterizing antibodies with specificity for a cleaved cis-acting hydrolase element sequence are well known to anyone of ordinary skill in the art. Such methods include the generation of antibody by immunizing laboratory animals resulting in either polyclonal sera or monoclonal antibodies by hybridoma technology, or the generation of antibodies by recombinant technologies, such as by screening large libraries of antibody fragments, obtained by the cloning of immune repertoires or the creation of fully or partly synthetic antibody libraries, in appropriate screening and/or selection systems, such as phage display. Furthermore, it is well known to one of ordinary skill to adapt existing methods for detecting, isolating and/or purifying proteins using antibodies to the methods of the present invention.

In one embodiment, the method is a method for isolating or purifying the recombinant Clostridial neurotoxin of the present invention, comprising the step of using an antibody in a process of separating polypeptides comprising a cleaved cis-acting hydrolase element sequence from compositions not comprising a cleaved cis-acting hydrolase element sequence.

In another embodiment, the method is a method for detecting the recombinant Clostridial neurotoxin of the present invention, comprising the step of using an antibody in a process of detecting polypeptides comprising a cleaved cis-acting hydrolase element sequence from compositions not comprising a cleaved cis-acting hydrolase element sequence.

In a further aspect, the invention relates to a pharmaceutical composition comprising the recombinant Clostridial neurotoxin of the present invention.

In one embodiment, the pharmaceutical composition of the present invention is for the treatment of a disease or condition taken from the list of: cervical dystonia (spasmodic torticollis), blepharospasm, severe primary axillary hyperhidrosis, achalasia, chronic focal painful neuropathies, migraine and other headache disorders, and cosmetic or aesthetic applications.

Additional indications where treatment with Botulinum neurotoxins is currently under investigation and where the pharmaceutical composition of the present invention may be used, include pediatric incontinence, incontinence due to overactive bladder, and incontinence due to neurogenic bladder, anal fissure, spastic disorders associated with injury or disease of the central nervous system including trauma, stroke, multiple sclerosis, Parkinson's disease, or cerebral palsy, focal dystonias affecting the limbs, face, jaw, or vocal cords, TMJ pain disorders, diabetic neuropathy, wound healing, excessive salivation, vocal cord dysfunction, reduction of the Masseter muscle for decreasing the size of the lower jaw, treatment and prevention of chronic headache and chronic musculoskeletal pain, treatment of snoring noise, assistance in weight loss by increasing the gastric emptying time.

### EXAMPLES

### Example 1: Expression of a recombinant Botulinum neurotoxin serotype A comprising a cis-acting hydrolase element in P. pastoris

WO 2006/017749 describes various approaches for designing and expressing nucleic acid constructs encoding Clostridial neurotoxins, including variants of Botulinum neurotoxin serotype A and of tetanus toxin, in different expression systems. Example 17 of WO 2006/017749 describe the design of the nucleic acid sequence for expression in P. pastoris, based on the preferred codon usage of that host organism (see Tables 1 and 2 in Example 1), the synthesis of the nucleic acid sequence, cloning into appropriate expression vectors, and expression.

By following the teaching of WO 2006/017749, a nucleic acid construct encoding a single contiguous amino acid sequence comprising, from N- to C-terminus, amino acids 2 to 438 of wild type Botulinum neurotoxin serotype A, followed by the cis-acting hydrolase element from FMDV, and amino acids 449 to 1296 of wild type Botulinum neurotoxin serotype A, can be designed, synthesized as shown in Example 2 of WO 2006/017749, and cloned and expressed as shown in Example 17 of WO 2006/017749. The full-length nucleic acid sequence can be found in SEQ ID NO: 5, and the full-length amino acid sequence of the single contiguous amino acid sequence can be found in SEQ ID NO: 6 (cis-acting hydrolase element from FMDV shown in bold/underline; *: cleavage during self-processing):

### SEQ ID NO: 5:

atgcctttcg ttaataagca gttcaattac aaagaccctg tgaacggagt ggatatcgct 60
tatattaaga ttcctaatgc tggtcaaatg caacctgtta aagccttcaa aatacataac 120
aagatctggg ttatccctga acgcgatact tttacgaacc ctgaggaagg tgatttgaac 180
cctccacctg aagctaaaca agttcctgtt tcgtattacg actccactta tttatcaacc 240
gacaatgaaa aggataacta cctgaaaggc gtcacaaagt tgttcgaaag aatttactca 300
actgatctgg gtcgtatgtt acttacaagt atcgtgagag gtattccatt ctggggggga 360
tctactatcg atacagaatt gaaagttatc gatactaatt gtatcaacgt tattcaaccc 420
gatggaagct accgttcaga ggaactaaat cttgttatca taggaccctc agctgacata 480

attcagtttg aatgcaagtc ctttggacat gaagttttga atttaacaag aaatggttac 540
ggttcgactc aatacattag attctctcca gatttcacat tcggttttga agagagttta 600
gaagtcgata caaacccatt gcttggggcc ggcaaattcg ctacagaccc cgccgtcact 660
ttggcacacg aacttattca tgctggtcac cgactatacg gtatagctat taacccaaat 720
agggttttta aagttaacac taacgcatat tacgaaatga gtggactcga agtaagtttt 780
gaagagctaa ggactttcgg gggtcatgat gctaagttta ttgattctct gcaagaaaat 840
gaatttaggc tttattacta taataagttt aaggatatcg cctccactct gaacaaggcc 900
aaatctatcg tgggcacaac cgccagcttg caatacatga aaaatgtgtt caaagagaag 960
tacctgttgt cagaagatac gagtggcaag tttagtgtag acaagcttaa gttcgacaaa 1020
ctttataaga tgctgactga gatttacacc gaagacaatt tcgtcaaatt tttcaaagtt 1080
ctcaaccgga agacctattt gaattttgac aaagccgtct ttaaaataaa cattgttcct 1140
aaagtcaact ataccattta tgacggattt aatttgagaa acaccaactt ggctgcaaac 1200
tttaacgggc aaaatacaga gattaataac atgaatttca cgaagttgaa gaacttcaca 1260
ggtttgttcg agttctacaa actactgtgt gtacggggca ttataacgtc caagcctgtg 1320
aaacagttgc taaactttga tttgctgaag ttggctggag atgtcgaatc taaccctgga 1380
cctgctttga acgatttgtg cattaaggta aataactggg acttattttt ctctccctct 1440
gaggataact tcaccaacga tttaaataaa ggcgaggaaa tcacttctga cacaaacatt 1500
gaggctgcag aggaaaatat atctcttgat cttattcagc aatactattt gacgtttaac 1560
tttgacaacg agccagagaa catttcaatc gaaaatctat cttcggatat tatcggccag 1620
ctggaactaa tgccaaatat tgagaggttc ccaaatggaa agaaatacga gcttgataag 1680
tatacgatgt ttcattactt acgtgcacaa gaattcgaac atggaaagtc aagaatcgct 1740
cttactaatt ccgttaacga ggctctgttg aatccttcaa gggtttatac ctttttctca 1800
tccgattacg ttaagaaagt caataaggct actgaggctg ccatgtttct cggatgggtg 1860
gaacagttgg tttatgactt tactgacgaa acctccgagg tttccactac cgataaaata 1920
gcagatatca ctataattat accctacatt ggacctgctc tcaatatcgg aaacatgtta 1980
tataaggatg actttgttgg tgctctaatt ttctccggcg ccgtaattct actggagttt 2040
attcccgaaa tagctatacc tgtcttggga acatttgctc tggtttcgta cattgctaat 2100
aaggttttga ctgtccaaac aattgataac gcactgtcaa agcgaaacga gaaatgggac 2160
gaggtatata agtatatagt aacgaactgg ctcgcaaagg tgaatactca aattgatctc 2220
attagaaaga aaatgaaaga agcattggaa aaccaagccg aggctacaaa agcgattata 2280
aattaccagt acaaccagta tactgaagag gaaaagaaca atattaattt taatatcgat 2340
gacttgagta gcaagctcaa tgaatccata aataaggcta tgattaacat aaacaaattc 2400
ttgaaccagt gtagcgttag ttatttgatg aactctatga taccatatgg ggttaagaga 2460
ctggaagatt ttgatgcctc gctgaaggat gcccttttga agtatattta cgacaatcga 2520
ggtaccctga tcggacaagt cgacagacta aaggacaagg tcaacaatac tttgtctacc 2580
gacatcccat ttcaactttc taaatatgtt gataaccaaa gattgctttc tactttcacc 2640

gaatacatta aaaatataat taataccagt attttgaatc tgagatatga gtctaaccac 2700
ttaatagatt taagtcgtta tgcctcgaag attaacattg ggtctaaagt gaactttgat 2760
ccgatcgaca agaaccagat ccagttgttt aatctcgaat cctcaaagat tgaggtcatt 2820
ttgaaaaatg caattgttta taacagcatg tacgaaaact tttccacctc tttttggatc 2880
agaattccaa agtactttaa ctcaatttca ttgaacaatg agtacactat tatcaattgt 2940
atggaaaata acagtggctg gaaagtttct ttaaattacg gtgagattat ctggacatta 3000
caagatactc aagaaatcaa acagagggtc gtatttaagt attctcaaat gattaatatt 3060
tccgactaca tcaatcgttg gatcttcgtg acaatcacta ataacagact gaataactca 3120
aaaatttata tcaacggtag attaattgat caaaaaccaa tttctaactt gggtaatatc 3180
cacgcttcta acaatatcat gttcaagtta gacggttgta gagatacaca ccgttatatc 3240
tggatcaagt acttcaatct tttcgataag gaactaaacg agaaggagat caaggactta 3300
tacgataatc aaagtaactc cggtattctt aaggattttt ggggtgacta cttgcagtat 3360
gacaaaccgt attacatgtt gaacttgtac gatccaaaca aatatgtgga cgttaataac 3420
gtaggaatta gaggatacat gtacttaaag ggtccaagag gatctgtaat gactacgaat 3480
atctacttaa actcctcact ctacagaggt accaaattta taattaagaa atatgcgtcc 3540
ggtaacaaag acaatatagt cagaaataac gatcgagttt acattaacgt tgtagtgaaa 3600
aataaggaat accgcctagc tactaacgca tctcaagcag gtgtcgagaa aatattgtcc 3660
gctcttgaaa ttccggacgt gggtaacctg tcgcaagtgg tcgttatgaa gagtaaaaat 3720
gaccagggaa ttaccaataa atgcaaaatg aaccttcagg ataataacgg taatgatatc 3780
ggatttatcg gttttcatca gttcaacaac attgcgaagt tagtcgcctc taactggtat 3840
aacagacaaa tcgagagaag ctctaggact ctaggttgta gctgggagtt cattccagtg 3900
gatgacggat ggggtgaacg tccattgtaa 3891

### SEQ ID NO: 6:

PFVNKQFNYKDPVNGVDIAYIKIPNAGQMQPVKAFKIHNKIWVIPERDTFTNPEEGDL
NPPPEAKQVPVSYYDSTYLSTDNEKDNYLKGVTKLFERIYSTDLGRMLLTSIVRGIPF
WGGSTIDTELKVIDTNCINVIQPDGSYRSEELNLVIIGPSADIIQFECKSFGHEVLNLTR
NGYGSTQYIRFSPDFTFGFEESLEVDTNPLLGAGKFATDPAVTLAHELIHAGHRLYGI
AINPNRVFKVNTNAYYEMSGLEVSFEELRTFGGHDAKFIDSLQENEFRLYYYNKFKDI
ASTLNKAKSIVGTTASLQYMKNVFKEKYLLSEDTSGKFSVDKLKFDKLYKMLTEIYT
EDNFVKFFKVLNRKTYLNFDKAVFKINIVPKVNYTIYDGFNLRNTNLAANFNGQNTEI
NNMNFTKLKNFTGLFEFYKLLCVRGIITSKPVKQLLNFDLLKLAGDVESNPG*PALN
DLCIKVNNWDLFFSPSEDNFTNDLNKGEEITSDTNIEAAEENISLDLIQQYYLTFNFDN
EPENISIENLSSDIIGQLELMPNIERFPNGKKYELDKYTMFHYLRAQEFEHGKSRIALTN

SVNEALLNPSRVYTFFSSDYVKKVNKATEAAMFLGWVEQLVYDFTDETSEVSTTDKI
ADITIIIPYIGPALNIGNMLYKDDFVGALIFSGAVILLEFIPEIAIPVLGTFALVSYIANKV
LTVQTIDNALSKRNEKWDEVYKYIVTNWLAKVNTQIDLIRKKMKEALENQAEATKA
IINYQYNQYTEEEKNNINFNIDDLSSKLNESINKAMININKFLNQCSVSYLMNSMIPYG
VKRLEDFDASLKDALLKYIYDNRGTLIGQVDRLKDKVNNTLSTDIPFQLSKYVDNQR
LLSTFTEYIKNIINTSILNLRYESNHLIDLSRYASKINIGSKVNFDPIDKNQIQLFNLESSK
IEVILKNAIVYNSMYENFSTSFWIRIPKYFNSISLNNEYTIINCMENNSGWKVSLNYGEI
IWTLQDTQEIKQRVVFKYSQMINISDYINRWIFVTITNNRLNNSKIYINGRLIDQKPISN
LGNIHASNNIMFKLDGCRDTHRYIWIKYFNLFDKELNEKEIKDLYDNQSNSGILKDFW
GDYLQYDKPYYMLNLYDPNKYVDVNNVGIRGYMYLKGPRGSVMTTNIYLNSSLYR
GTKFIIKKYASGNKDNIVRNNDRVYINVVVKNKEYRLATNASQAGVEKILSALEIPDV
GNLSQVVVMKSKNDQGITNKCKMNLQDNNGNDIGFIGFHQFNNIAKLVASNWYNR
QIERSSRTLGCSWEFIPVDDGWGERPL

After expression, the expression products are isolated, identified and characterized as shown in Example 17 of WO 2006/017749, showing that under nonreducing conditions a protein of approx. 150 kDa is can be detected, while under reducing conditions two proteins of 100 kDa and 50 kDa, corresponding to the heavy and light chain, respectively, can be detected, indicating the correct formation of a disulfide-bonded heterodimeric botulinum neurotoxin.

### Example 2: Expression of a recombinant Botulinum neurotoxin serotype A comprising a cis-acting hydrolase element in insect cells

WO 2006/017749 describes various approaches for designing and expressing nucleic acid constructs encoding Clostridial neurotoxins, including variants of Botulinum neurotoxin serotype A and of tetanus toxin, in different expression systems. Example 20 of WO 2006/017749 describe the design of the nucleic acid sequence for expression in insect cells, based on the preferred codon usage of that host organism (see Tables 1 and 2 in Example 1), the synthesis of the nucleic acid sequence, cloning into appropriate expression vectors using a baculovirus expression system, and expression.

By following the teaching of WO 2006/017749, a nucleic acid construct encoding a single contiguous amino acid sequence comprising, from N- to C-terminus, amino acids 2 to 438 of wild type Botulinum neurotoxin serotype A, followed by the cis-acting hydrolase element from Thosea asigna virus (TaV), and amino acids 449 to 1296 of wild type Botulinum neurotoxin serotype A, can be designed, synthesized as shown in Example 2 of WO 2006/017749, and cloned and expressed as shown in Example 20 of WO 2006/017749. The full-length amino acid sequence of the single contiguous amino acid sequence can be found in SEQ ID NO: 7 (cis-acting hydrolase element from TaV shown in bold/underline; *: cleavage during self-processing):

### SEQ ID NO: 7:

PFVNKQFNYKDPVNGVDIAYIKIPNAGQMQPVKAFKIHNKIWVIPERDTFTNPEEGDL
NPPPEAKQVPVSYYDSTYLSTDNEKDNYLKGVTKLFERIYSTDLGRMLLTSIVRGIPF
WGGSTIDTELKVIDTNCINVIQPDGSYRSEELNLVIIGPSADIIQFECKSFGHEVLNLTR
NGYGSTQYIRFSPDFTFGFEESLEVDTNPLLGAGKFATDPAVTLAHELIHAGHRLYGI
AINPNRVFKVNTNAYYEMSGLEVSFEELRTFGGHDAKFIDSLQENEFRLYYYNKFKDI
ASTLNKAKSIVGTTASLQYMKNVFKEKYLLSEDTSGKFSVDKLKFDKLYKMLTEIYT
EDNFVKFFKVLNRKTYLNFDKAVFKINIVPKVNYTIYDGFNLRNTNLAANFNGQNTEI
NNMNFTKLKNFTGLFEFYKLLCVRGIITSKEGRGSLLTCGDVEENPG*PALNDLCIK
VNNWDLFFSPSEDNFTNDLNKGEEITSDTNIEAAEENISLDLIQQYYLTFNFDNEPENIS
IENLSSDIIGQLELMPNIERFPNGKKYELDKYTMFHYLRAQEFEHGKSRIALTNSVNEA
LLNPSRVYTFFSSDYVKKVNKATEAAMFLGWVEQLVYDFTDETSEVSTTDKIADITIII
PYIGPALNIGNMLYKDDFVGALIFSGAVILLEFIPEIAIPVLGTFALVSYIANKVLTVQTI
DNALSKRNEKWDEVYKYIVTNWLAKVNTQIDLIRKKMKEALENQAEATKAIINYQY
NQYTEEEKNNINFNIDDLSSKLNESINKAMININKFLNQCSVSYLMNSMIPYGVKRLE
DFDASLKDALLKYIYDNRGTLIGQVDRLKDKVNNTLSTDIPFQLSKYVDNQRLLSTFT
EYIKNIINTSILNLRYESNHLIDLSRYASKINIGSKVNFDPIDKNQIQLFNLESSKIEVILK
NAIVYNSMYENFSTSFWIRIPKYFNSISLNNEYTIINCMENNSGWKVSLNYGEIIWTLQ
DTQEIKQRVVFKYSQMINISDYINRWIFVTITNNRLNNSKIYINGRLIDQKPISNLGNIH
ASNNIMFKLDGCRDTHRYIWIKYFNLFDKELNEKEIKDLYDNQSNSGILKDFWGDYL
QYDKPYYMLNLYDPNKYVDVNNVGIRGYMYLKGPRGSVMTTNIYLNSSLYRGTKFI
IKKYASGNKDNIVRNNDRVYINVVVKNKEYRLATNASQAGVEKILSALEIPDVGNLS

QVVVMKSKNDQGITNKCKMNLQDNNGNDIGFIGFHQFNNIAKLVASNWYNRQIERS
SRTLGCSWEFIPVDDGWGERPL

After expression, the expression products are isolated, identified and characterized as shown in Example 20 of WO 2006/017749, showing that under nonreducing conditions a protein of approx. 150 kDa is can be detected, while under reducing conditions two proteins of 100 kDa and 50 kDa, corresponding to the heavy and light chain, respectively, can be detected, indicating the correct formation of a disulfide-bonded heterodimeric botulinum neurotoxin.

### Example 3: Expression of a recombinant tetanus toxin comprising a cis-acting hydrolase element in P. pastoris

WO 2006/017749 describes various approaches for designing and expressing nucleic acid constructs encoding Clostridial neurotoxins, including variants of Botulinum neurotoxin serotype A and of tetanus toxin, in different expression systems. Example 17 of WO 2006/017749 describe the design of the nucleic acid sequence for expression in P. pastoris, based on the preferred codon usage of that host organism (see Tables 1 and 2 in Example 1), the synthesis of the nucleic acid sequence, cloning into appropriate expression vectors, and expression.

By following the teaching of WO 2006/017749, a nucleic acid construct encoding a single contiguous amino acid sequence comprising, from N- to C-terminus, amino acids 2 to 445 of wild type tetanus toxin, followed by the cis-acting hydrolase element from FMDV, and amino acids 461 to 1314 of wild type tetanus toxin, can be designed, synthesized as shown in Example 2 of WO 2006/017749, and cloned and expressed as shown in Example 17 of WO 2006/017749. The full-length amino acid sequence of the single contiguous amino acid sequence can be found in SEQ ID NO: 8 (cis-acting hydrolase element from FMDV shown in bold/underline; *: cleavage during self-processing):

### SEQ ID NO: 8:

PITINNFRY SDPVNNDTII MMEPPYCKGL DIYYKAFKIT DRIWIVPERY
EFGTKPEDFN PPSSLIEGAS EYYDPNYLRT DSDKDRFLQT MVKLFNRIKN

NVAGEALLDK IINAIPYLGN SYSLLDKFDT NSNSVSFNLL EQDPSGATTK
SAMLTNLIIF GPGPVLNKNE VRGIVLRVDN KNYFPCRDGF GSIMQMAFCP
EYVPTFDNVI ENITSLTIGK SKYFQDPALL LMHELIHVLH GLYGMQVSSH
EIIPSKQEIY MQHTYPISAE ELFTFGGQDA NLISIDIKND LYEKTLNDYK
AIANKLSQVT SCNDPNIDID SYKQIYQQKY QFDKDSNGQY IVNEDKFQIL
YNSIMYGFTE IELGKKFNIK TRLSYFSMNH DPVKIPNLLD DTIYNDTEGF
NIESKDLKSE YKGQNMRVNT NAFRNVDGSG LVSKLIGLCK
KIIPPPVKQLLNFDLLKLAGDVESNPG*PDLGGELCIKI KNEDLTFIAE
KNSFSEEPFQ DEIVSYNTKN KPLNFNYSLD KIIVDYNLQS KITLPNDRTT
PVTKGIPYAP EYKSNAASTI EIHNIDDNTI YQYLYAQKSP TTLQRITMTN
SVDDALINST KIYSYFPSVI SKVNQGAQGI LFLQWVRDII DDFTNESSQK
TTIDKISDVS TIVPYIGPAL NIVKQGYEGN FIGALETTGV VLLLEYIPEI TLPVIAALSI
AESSTQKEKI IKTIDNFLEK RYEKWIEVYK LVKAKWLGTV NTQFQKRSYQ
MYRSLEYQVD AIKKIIDYEY KIYSGPDKEQ IADEINNLKN KLEEKANKAM
ININIFMRES SRSFLVNQMI NEAKKQLLEF DTQSKNILMQ YIKANSKFIG
ITELKKLESK INKVFSTPIP FSYSKNLDCW VDNEEDIDVI LKKSTILNLD INNDIISDIS
GFNSSVITYP DAQLVPGING KAIHLVNNES SEVIVHKAMD IEYNDMFNNF
TVSFWLRVPK VSASHLEQYG TNEYSIISSM KKHSLSIGSG WSVSLKGNNL
IWTLKDSAGE VRQITFRDLP DKFNAYLANK WVFITITNDR LSSANLYING
VLMGSAEITG LGAIREDNNI TLKLDRCNNN NQYVSIDKFR IFCKALNPKE
IEKLYTSYLS ITFLRDFWGN PLRYDTEYYL IPVASSSKDV QLKNITDYMY
LTNAPSYTNG KLNIYYRRLY NGLKFIIKRY TPNNEIDSFV KSGDFIKLYV
SYNNNEHIVG YPKDGNAFNN LDRILRVGYN APGIPLYKKM EAVKLRDLKT
YSVQLKLYDD KNASLGLVGT HNGQIGNDPN RDILIASNWY FNHLKDKILG
CDWYFVPTDE GWTND

After expression, the expression products are isolated, identified and characterized as shown in Example 17 of WO 2006/017749, showing that under nonreducing conditions a protein of approx. 150 kDa is can be detected, while under reducing conditions two proteins of 100 kDa and 50 kDa, corresponding to the heavy and light chain, respectively, can be detected, indicating the correct formation of a disulfide-bonded heterodimeric botulinum neurotoxin.

### SEQ ID NO: 1:

PFVNKQFNYKDPVNGVDIAYIKIPNAGQMQPVKAFKIHNKIWVIPERDTFTNPEEGDL
NPPPEAKQVPVSYYDSTYLSTDNEKDNYLKGVTKLFERIYSTDLGRMLLTSIVRGIPF
WGGSTIDTELKVIDTNCINVIQPDGSYRSEELNLVIIGPSADIIQFECKSFGHEVLNLTR
NGYGSTQYIRFSPDFTFGFEESLEVDTNPLLGAGKFATDPAVTLAHELIHAGHRLYGI
AINPNRVFKVNTNAYYEMSGLEVSFEELRTFGGHDAKFIDSLQENEFRLYYYNKFKDI
ASTLNKAKSIVGTTASLQYMKNVFKEKYLLSEDTSGKFSVDKLKFDKLYKMLTEIYT
EDNFVKFFKVLNRKTYLNFDKAVFKINIVPKVNYTIYDGFNLRNTNLAANFNGQNTEI
NNMNFTKLKNFTGLFEFYKLLCVRGIITSKPVKQLLNFDLLKLAGDVESNPG

### SEQ ID NO: 2:

PALNDLCIKVNNWDLFFSPSEDNFTNDLNKGEEITSDTNIEAAEENISLDLIQQYYLTF
NFDNEPENISIENLSSDIIGQLELMPNIERFPNGKKYELDKYTMFHYLRAQEFEHGKSRI
ALTNSVNEALLNPSRVYTFFSSDYVKKVNKATEAAMFLGWVEQLVYDFTDETSEVS
TTDKIADITIIIPYIGPALNIGNMLYKDDFVGALIFSGAVILLEFIPEIAIPVLGTFALVSYI
ANKVLTVQTIDNALSKRNEKWDEVYKYIVTNWLAKVNTQIDLIRKKMKEALENQAE
ATKAIINYQYNQYTEEEKNNINFNIDDLSSKLNESINKAMININKFLNQCSVSYLMNS
MIPYGVKRLEDFDASLKDALLKYIYDNRGTLIGQVDRLKDKVNNTLSTDIPFQLSKY
VDNQRLLSTFTEYIKNIINTSILNLRYESNHLIDLSRYASKINIGSKVNFDPIDKNQIQLF
NLESSKIEVILKNAIVYNSMYENFSTSFWIRIPKYFNSISLNNEYTIINCMENNSGWKVS
LNYGEIIWTLQDTQEIKQRVVFKYSQMINISDYINRWIFVTITNNRLNNSKIYINGRLID
QKPISNLGNIHASNNIMFKLDGCRDTHRYIWIKYFNLFDKELNEKEIKDLYDNQSNSGI
LKDFWGDYLQYDKPYYMLNLYDPNKYVDVNNVGIRGYMYLKGPRGSVMTTNIYL
NSSLYRGTKFIIKKYASGNKDNIVRNNDRVYINVVVKNKEYRLATNASQAGVEKILS
ALEIPDVGNLSQVVVMKSKNDQGITNKCKMNLQDNNGNDIGFIGFHQFNNIAKLVAS
NWYNRQIERSSRTLGCSWEFIPVDDGWGERPL

### SEQ ID NO: 3:

PITINNFRY SDPVNNDTII MMEPPYCKGL DIYYKAFKIT DRIWIVPERY
EFGTKPEDFN PPSSLIEGAS EYYDPNYLRT DSDKDRFLQT MVKLFNRIKN
NVAGEALLDK IINAIPYLGN SYSLLDKFDT NSNSVSFNLL EQDPSGATTK
SAMLTNLIIF GPGPVLNKNE VRGIVLRVDN KNYFPCRDGF GSIMQMAFCP
EYVPTFDNVI ENITSLTIGK SKYFQDPALL LMHELIHVLH GLYGMQVSSH
EIIPSKQEIY MQHTYPISAE ELFTFGGQDA NLISIDIKND LYEKTLNDYK
AIANKLSQVT SCNDPNIDID SYKQIYQQKY QFDKDSNGQY IVNEDKFQIL
YNSIMYGFTE IELGKKFNIK TRLSYFSMNH DPVKIPNLLD DTIYNDTEGF
NIESKDLKSE YKGQNMRVNT NAFRNVDGSG LVSKLIGLCK
KIIPPPVKQLLNFDLLKLAGDVESNPG

### SEQ ID NO: 4:

PDLGGELCIKI KNEDLTFIAE KNSFSEEPFQ DEIVSYNTKN KPLNFNYSLD
KIIVDYNLQS KITLPNDRTT PVTKGIPYAP EYKSNAASTI EIHNIDDNTI
YQYLYAQKSP TTLQRITMTN SVDDALINST KIYSYFPSVI SKVNQGAQGI
LFLQWVRDII DDFTNESSQK TTIDKISDVS TIVPYIGPAL NIVKQGYEGN
FIGALETTGV VLLLEYIPEI TLPVIAALSI AESSTQKEKI IKTIDNFLEK
RYEKWIEVYK LVKAKWLGTV NTQFQKRSYQ MYRSLEYQVD AIKKIIDYEY
KIYSGPDKEQ IADEINNLKN KLEEKANKAM ININIFMRES SRSFLVNQMI
NEAKKQLLEF DTQSKNILMQ YIKANSKFIG ITELKKLESK INKVFSTPIP
FSYSKNLDCW VDNEEDIDVI LKKSTILNLD INNDIISDIS GFNSSVITYP
DAQLVPGING KAIHLVNNES SEVIVHKAMD IEYNDMFNNF TVSFWLRVPK
VSASHLEQYG TNEYSIISSM KKHSLSIGSG WSVSLKGNNL IWTLKDSAGE
VRQITFRDLP DKFNAYLANK WVFITITNDR LSSANLYING VLMGSAEITG
LGAIREDNNI TLKLDRCNNN NQYVSIDKFR IFCKALNPKE IEKLYTSYLS
ITFLRDFWGN PLRYDTEYYL IPVASSSKDV QLKNITDYMY LTNAPSYTNG
KLNIYYRRLY NGLKFIIKRY TPNNEIDSFV KSGDFIKLYV SYNNNEHIVG
YPKDGNAFNN LDRILRVGYN APGIPLYKKM EAVKLRDLKT YSVQLKLYDD
KNASLGLVGT HNGQIGNDPN RDILIASNWY FNHLKDKILG CDWYFVPTDE
GWTND

### SEQ ID NO: 5:

atgcctttcg ttaataagca gttcaattac aaagaccctg tgaacggagt ggatatcgct 60
tatattaaga ttcctaatgc tggtcaaatg caacctgtta aagccttcaa aatacataac 120
aagatctggg ttatccctga acgcgatact tttacgaacc ctgaggaagg tgatttgaac 180
cctccacctg aagctaaaca agttcctgtt tcgtattacg actccactta tttatcaacc 240
gacaatgaaa aggataacta cctgaaaggc gtcacaaagt tgttcgaaag aatttactca 300
actgatctgg gtcgtatgtt acttacaagt atcgtgagag gtattccatt ctggggggga 360
tctactatcg atacagaatt gaaagttatc gatactaatt gtatcaacgt tattcaaccc 420
gatggaagct accgttcaga ggaactaaat cttgttatca taggaccctc agctgacata 480
attcagtttg aatgcaagtc ctttggacat gaagttttga atttaacaag aaatggttac 540
ggttcgactc aatacattag attctctcca gatttcacat tcggttttga agagagttta 600
gaagtcgata caaacccatt gcttggggcc ggcaaattcg ctacagaccc cgccgtcact 660
ttggcacacg aacttattca tgctggtcac cgactatacg gtatagctat taacccaaat 720
agggttttta aagttaacac taacgcatat tacgaaatga gtggactcga agtaagtttt 780
gaagagctaa ggactttcgg gggtcatgat gctaagttta ttgattctct gcaagaaaat 840
gaatttaggc tttattacta taataagttt aaggatatcg cctccactct gaacaaggcc 900
aaatctatcg tgggcacaac cgccagcttg caatacatga aaaatgtgtt caaagagaag 960
tacctgttgt cagaagatac gagtggcaag tttagtgtag acaagcttaa gttcgacaaa 1020
ctttataaga tgctgactga gatttacacc gaagacaatt tcgtcaaatt tttcaaagtt 1080
ctcaaccgga agacctattt gaattttgac aaagccgtct ttaaaataaa cattgttcct 1140
aaagtcaact ataccattta tgacggattt aatttgagaa acaccaactt ggctgcaaac 1200
tttaacgggc aaaatacaga gattaataac atgaatttca cgaagttgaa gaacttcaca 1260
ggtttgttcg agttctacaa actactgtgt gtacggggca ttataacgtc caagcctgtg 1320
aaacagttgc taaactttga tttgctgaag ttggctggag atgtcgaatc taaccctgga 1380
cctgctttga acgatttgtg cattaaggta aataactggg acttattttt ctctccctct 1440
gaggataact tcaccaacga tttaaataaa ggcgaggaaa tcacttctga cacaaacatt 1500
gaggctgcag aggaaaatat atctcttgat cttattcagc aatactattt gacgtttaac 1560
tttgacaacg agccagagaa catttcaatc gaaaatctat cttcggatat tatcggccag 1620
ctggaactaa tgccaaatat tgagaggttc ccaaatggaa agaaatacga gcttgataag 1680
tatacgatgt ttcattactt acgtgcacaa gaattcgaac atggaaagtc aagaatcgct 1740
cttactaatt ccgttaacga ggctctgttg aatccttcaa gggtttatac ctttttctca 1800
tccgattacg ttaagaaagt caataaggct actgaggctg ccatgtttct cggatgggtg 1860
gaacagttgg tttatgactt tactgacgaa acctccgagg tttccactac cgataaaata 1920
gcagatatca ctataattat accctacatt ggacctgctc tcaatatcgg aaacatgtta 1980

tataaggatg actttgttgg tgctctaatt ttctccggcg ccgtaattct actggagttt 2040
attcccgaaa tagctatacc tgtcttggga acatttgctc tggtttcgta cattgctaat 2100
aaggttttga ctgtccaaac aattgataac gcactgtcaa agcgaaacga gaaatgggac 2160
gaggtatata agtatatagt aacgaactgg ctcgcaaagg tgaatactca aattgatctc 2220
attagaaaga aaatgaaaga agcattggaa aaccaagccg aggctacaaa agcgattata 2280
aattaccagt acaaccagta tactgaagag gaaaagaaca atattaattt taatatcgat 2340
gacttgagta gcaagctcaa tgaatccata aataaggcta tgattaacat aaacaaattc 2400
ttgaaccagt gtagcgttag ttatttgatg aactctatga taccatatgg ggttaagaga 2460
ctggaagatt ttgatgcctc gctgaaggat gcccttttga agtatattta cgacaatcga 2520
ggtaccctga tcggacaagt cgacagacta aaggacaagg tcaacaatac tttgtctacc 2580
gacatcccat ttcaactttc taaatatgtt gataaccaaa gattgctttc tactttcacc 2640
gaatacatta aaaatataat taataccagt attttgaatc tgagatatga gtctaaccac 2700
ttaatagatt taagtcgtta tgcctcgaag attaacattg ggtctaaagt gaactttgat 2760
ccgatcgaca agaaccagat ccagttgttt aatctcgaat cctcaaagat tgaggtcatt 2820
ttgaaaaatg caattgttta taacagcatg tacgaaaact tttccacctc tttttggatc 2880
agaattccaa agtactttaa ctcaatttca ttgaacaatg agtacactat tatcaattgt 2940
atggaaaata acagtggctg gaaagtttct ttaaattacg gtgagattat ctggacatta 3000
caagatactc aagaaatcaa acagagggtc gtatttaagt attctcaaat gattaatatt 3060
tccgactaca tcaatcgttg gatcttcgtg acaatcacta ataacagact gaataactca 3120
aaaatttata tcaacggtag attaattgat caaaaaccaa tttctaactt gggtaatatc 3180
cacgcttcta acaatatcat gttcaagtta gacggttgta gagatacaca ccgttatatc 3240
tggatcaagt acttcaatct tttcgataag gaactaaacg agaaggagat caaggactta 3300
tacgataatc aaagtaactc cggtattctt aaggattttt ggggtgacta cttgcagtat 3360
gacaaaccgt attacatgtt gaacttgtac gatccaaaca aatatgtgga cgttaataac 3420
gtaggaatta gaggatacat gtacttaaag ggtccaagag gatctgtaat gactacgaat 3480
atctacttaa actcctcact ctacagaggt accaaattta taattaagaa atatgcgtcc 3540
ggtaacaaag acaatatagt cagaaataac gatcgagttt acattaacgt tgtagtgaaa 3600
aataaggaat accgcctagc tactaacgca tctcaagcag gtgtcgagaa aatattgtcc 3660
gctcttgaaa ttccggacgt gggtaacctg tcgcaagtgg tcgttatgaa gagtaaaaat 3720
gaccagggaa ttaccaataa atgcaaaatg aaccttcagg ataataacgg taatgatatc 3780
ggatttatcg gttttcatca gttcaacaac attgcgaagt tagtcgcctc taactggtat 3840
aacagacaaa tcgagagaag ctctaggact ctaggttgta gctgggagtt cattccagtg 3900
gatgacggat ggggtgaacg tccattgtaa 3891

### SEQ ID NO: 6:

PFVNKQFNYKDPVNGVDIAYIKIPNAGQMQPVKAFKIHNKIWVIPERDTFTNPEEGDL
NPPPEAKQVPVSYYDSTYLSTDNEKDNYLKGVTKLFERIYSTDLGRMLLTSIVRGIPF
WGGSTIDTELKVIDTNCINVIQPDGSYRSEELNLVIIGPSADIIQFECKSFGHEVLNLTR
NGYGSTQYIRFSPDFTFGFEESLEVDTNPLLGAGKFATDPAVTLAHELIHAGHRLYGI
AINPNRVFKVNTNAYYEMSGLEVSFEELRTFGGHDAKFIDSLQENEFRLYYYNKFKDI
ASTLNKAKSIVGTTASLQYMKNVFKEKYLLSEDTSGKFSVDKLKFDKLYKMLTEIYT
EDNFVKFFKVLNRKTYLNFDKAVFKINIVPKVNYTIYDGFNLRNTNLAANFNGQNTEI
NNMNFTKLKNFTGLFEFYKLLCVRGIITSKPVKQLLNFDLLKLAGDVESNPGPALN
DLCIKVNNWDLFFSPSEDNFTNDLNKGEEITSDTNIEAAEENISLDLIQQYYLTFNFDN
EPENISIENLSSDIIGQLELMPNIERFPNGKKYELDKYTMFHYLRAQEFEHGKSRIALTN
SVNEALLNPSRVYTFFSSDYVKKVNKATEAAMFLGWVEQLVYDFTDETSEVSTTDKI
ADITIIIPYIGPALNIGNMLYKDDFVGALIFSGAVILLEFIPEIAIPVLGTFALVSYIANKV
LTVQTIDNALSKRNEKWDEVYKYIVTNWLAKVNTQIDLIRKKMKEALENQAEATKA
IINYQYNQYTEEEKNNINFNIDDLSSKLNESINKAMININKFLNQCSVSYLMNSMIPYG
VKRLEDFDASLKDALLKYIYDNRGTLIGQVDRLKDKVNNTLSTDIPFQLSKYVDNQR
LLSTFTEYIKNIINTSILNLRYESNHLIDLSRYASKINIGSKVNFDPIDKNQIQLFNLESSK
IEVILKNAIVYNSMYENFSTSFWIRIPKYFNSISLNNEYTIINCMENNSGWKVSLNYGEI
IWTLQDTQEIKQRVVFKYSQMINISDYINRWIFVTITNNRLNNSKIYINGRLIDQKPISN
LGNIHASNNIMFKLDGCRDTHRYIWIKYFNLFDKELNEKEIKDLYDNQSNSGILKDFW
GDYLQYDKPYYMLNLYDPNKYVDVNNVGIRGYMYLKGPRGSVMTTNIYLNSSLYR
GTKFIIKKYASGNKDNIVRNNDRVYINVVVKNKEYRLATNASQAGVEKILSALEIPDV
GNLSQVVVMKSKNDQGITNKCKMNLQDNNGNDIGFIGFHQFNNIAKLVASNWYNR
QIERSSRTLGCSWEFIPVDDGWGERPL

### SEQ ID NO: 7:

PFVNKQFNYKDPVNGVDIAYIKIPNAGQMQPVKAFKIHNKIWVIPERDTFTNPEEGDL
NPPPEAKQVPVSYYDSTYLSTDNEKDNYLKGVTKLFERIYSTDLGRMLLTSIVRGIPF
WGGSTIDTELKVIDTNCINVIQPDGSYRSEELNLVIIGPSADIIQFECKSFGHEVLNLTR
NGYGSTQYIRFSPDFTFGFEESLEVDTNPLLGAGKFATDPAVTLAHELIHAGHRLYGI
AINPNRVFKVNTNAYYEMSGLEVSFEELRTFGGHDAKFIDSLQENEFRLYYYNKFKDI
ASTLNKAKSIVGTTASLQYMKNVFKEKYLLSEDTSGKFSVDKLKFDKLYKMLTEIYT

EDNFVKFFKVLNRKTYLNFDKAVFKINIVPKVNYTIYDGFNLRNTNLAANFNGQNTEI
NNMNFTKLKNFTGLFEFYKLLCVRGIITSKEGRGSLLTCGDVEENPGPALNDLCIKV
NNWDLFFSPSEDNFTNDLNKGEEITSDTNIEAAEENISLDLIQQYYLTFNFDNEPENISI
ENLSSDIIGQLELMPNIERFPNGKKYELDKYTMFHYLRAQEFEHGKSRIALTNSVNEA
LLNPSRVYTFFSSDYVKKVNKATEAAMFLGWVEQLVYDFTDETSEVSTTDKIADITIII
PYIGPALNIGNMLYKDDFVGALIFSGAVILLEFIPEIAIPVLGTFALVSYIANKVLTVQTI
DNALSKRNEKWDEVYKYIVTNWLAKVNTQIDLIRKKMKEALENQAEATKAIINYQY
NQYTEEEKNNINFNIDDLSSKLNESINKAMININKFLNQCSVSYLMNSMIPYGVKRLE
DFDASLKDALLKYIYDNRGTLIGQVDRLKDKVNNTLSTDIPFQLSKYVDNQRLLSTFT
EYIKNIINTSILNLRYESNHLIDLSRYASKINIGSKVNFDPIDKNQIQLFNLESSKIEVILK
NAIVYNSMYENFSTSFWIRIPKYFNSISLNNEYTIINCMENNSGWKVSLNYGEIIWTLQ
DTQEIKQRVVFKYSQMINISDYINRWIFVTITNNRLNNSKIYINGRLIDQKPISNLGNIH
ASNNIMFKLDGCRDTHRYIWIKYFNLFDKELNEKEIKDLYDNQSNSGILKDFWGDYL
QYDKPYYMLNLYDPNKYVDVNNVGIRGYMYLKGPRGSVMTTNIYLNSSLYRGTKFI
IKKYASGNKDNIVRNNDRVYINVVVKNKEYRLATNASQAGVEKILSALEIPDVGNLS
QVVVMKSKNDQGITNKCKMNLQDNNGNDIGFIGFHQFNNIAKLVASNWYNRQIERS
SRTLGCSWEFIPVDDGWGERPL

### SEQ ID NO: 8:

PITINNNFRY SDPVNNDTII MMEPPYCKGL DIYYKAFKIT DRIWIVPERY
EFGTKPEDFN PPSSLIEGAS EYYDPNYLRT DSDKDRFLQT MVKLFNRIKN
NVAGEALLDK IINAIPYLGN SYSLLDKFDT NSNSVSFNLL EQDPSGATTK
SAMLTNLIIF GPGPVLNKNE VRGIVLRVDN KNYFPCRDGF GSIMQMAFCP
EYVPTFDNVI ENITSLTIGK SKYFQDPALL LMHELIHVLH GLYGMQVSSH
EIIPSKQEIY MQHTYPISAE ELFTFGGQDA NLISIDIKND LYEKTLNDYK
AIANKLSQVT SCNDPNIDID SYKQIYQQKY QFDKDSNGQY IVNEDKFQIL
YNSIMYGFTE IELGKKFNIK TRLSYFSMNH DPVKIPNLLD DTIYNDTEGF
NIESKDLKSE YKGQNMRVNT NAFRNVDGSG LVSKLIGLCK
KIIPPPVKQLLNFDLLKLAGDVESNPGPDLGGELCIKI KNEDLTFIAE KNSFSEEPFQ
DEIVSYNTKN KPLNFNYSLD KIIVDYNLQS KITLPNDRTT PVTKGIPYAP
EYKSNAASTI EIHNIDDNTI YQYLYAQKSP TTLQRITMTN SVDDALINST
KIYSYFPSVI SKVNQGAQGI LFLQWVRDII DDFTNESSQK TTIDKISDVS

TIVPYIGPAL NIVKQGYEGN FIGALETTGV VLLLEYIPEI TLPVIAALSI AESSTQKEKI
IKTIDNFLEK RYEKWIEVYK LVKAKWLGTV NTQFQKRSYQ MYRSLEYQVD
AIKKIIDYEY KIYSGPDKEQ IADEINNLKN KLEEKANKAM ININIFMRES
SRSFLVNQMI NEAKKQLLEF DTQSKNILMQ YIKANSKFIG ITELKKLESK
INKVFSTPIP FSYSKNLDCW VDNEEDIDVI LKKSTILNLD INNDIISDIS GFNSSVITYP
DAQLVPGING KAIHLVNNES SEVIVHKAMD IEYNDMFNNF TVSFWLRVPK
VSASHLEQYG TNEYSIISSM KKHSLSIGSG WSVSLKGNNL IWTLKDSAGE
VRQITFRDLP DKFNAYLANK WVFITITNDR LSSANLYING VLMGSAEITG
LGAIREDNNI TLKLDRCNNN NQYVSIDKFR IFCKALNPKE IEKLYTSYLS
ITFLRDFWGN PLRYDTEYYL IPVASSSKDV QLKNITDYMY LTNAPSYTNG
KLNIYYRRLY NGLKFIIKRY TPNNEIDSFV KSGDFIKLYV SYNNNEHIVG
YPKDGNAFNN LDRILRVGYN APGIPLYKKM EAVKLRDLKT YSVQLKLYDD
KNASLGLVGT HNGQIGNDPN RDILIASNWY FNHLKDKILG CDWYFVPTDE
GWTND

## Claims

1. A recombinant Clostridial neurotoxin comprising: (i) a functionally active Clostridial neurotoxin light chain; and (ii) a functionally active Clostridial neurotoxin heavy chain, **characterized in that** said light chain comprises a cleaved cis-acting hydrolase element at the C-terminus of said light chain, and said heavy chain comprises a proline residue located at the N-terminus of said heavy chain.

2. The recombinant Clostridial neurotoxin of claim 1, which is a functional homologue of a Clostridium botulinum neurotoxin taken from the list of: Clostridium botulinum neurotoxin serotype A, B, C, D, E, F, and G.

3. The recombinant Clostridial neurotoxin of claim 1 or 2, wherein said light chain consists of (a) amino acid residues 2 to 438 of Clostridium botulinum neurotoxin serotype A; or of (b) a functionally active variant of the light chain defined in (a), fused to a cleaved cis-acting hydrolase element at the C-terminus of said light chain.

4. The recombinant Clostridial neurotoxin of claim 1 or 2, wherein said heavy chain consists of an N-terminal proline linked to (a) amino acid residues 449 to 1296 of Clostridium botulinum neurotoxin serotype A; or (b) a functionally active variant of the heavy chain defined in (a), fused to a cleaved cis-acting hydrolase element at the C-terminus of said light chain.

5. The recombinant Clostridial neurotoxin of any one of claim 1 to 4, wherein said light chain has the amino acid sequence as found in SEQ ID NO: 1, and wherein said heavy chain has the amino acid sequence as found in SEQ ID NO: 2.

6. The recombinant Clostridial neurotoxin of any one of claims 1 to 5, wherein said cleaved cis-acting hydrolase element is comprised in a cis-acting hydrolase element from (i) a virus taken from the list of: EMC-B, EMC-D, EMC-PV21, TME-GD7, TME-DA, DME-BEAN, Theiler's-Like Virus, Ljungan virus (174F), Ljungan virus (145SL), Ljungan virus (87-012), Ljungan virus (M1146), FMD-A10, FMD-A12, FMD-C1, FMD-O1G FMD-O1K FMD-O (Taiwan), FMD-O/SK, FMD-SAT3, FMD-SAT2, ERAV, ERBV, ERV-3, PTV-1, PTV-2, PTV-3, PTV-4, PTV-5, PTV-6, PTV-7, PTV-8, PTV-9, PTV-10, PTV-11, CrPV, DCV, ABPV, ABPV isolate Poland 1, ABPV isolate Hungary 1, IFV, TaV, EEV, KBV, PnPV (a), PnPV (b), Ectropis oblique picorna-like virus (a), Ectropis oblique picorna-like virus (b), Providence virus (a), Providence virus (b), Providence virus (c), Bovine Rotavirus, Porcine Rotavirus, Human Rotavirus, Bombyx mori, Lymantria dispar, Dendrolimus punctatus; (ii) a Trypansoma spp. Repeated Sequence taken from the list of: T. brucei TSR1, T. brucei (CAB95325.1), T. brucei (CAB95342.1), T. brucei (CAB95559.1), T. cruzi AP Endonuclease, or (iii) a cis-acting hydrolase element that is a functional variant or a cis-acting hydrolase element listed in (i) or (ii).

7. A nucleic acid sequence encoding the recombinant Clostridial neurotoxin of any one of claims 1 to 6 comprising (i) a nucleic acid sequence encoding a functionally active Clostridial neurotoxin light chain, (ii) a nucleic acid sequence encoding a cis-acting hydrolase element, and (iii) a nucleic acid sequence encoding a functionally active Clostridial neurotoxin heavy chain.

8. The nucleic acid sequence of claim 7, wherein said nucleic acid has the sequence as found in SEQ ID NO: 5.

9. A method for obtaining the nucleic acid sequence of claim 7, comprising the step of inserting of a nucleic acid sequence encoding a cis-acting hydrolase element into a nucleic acid sequence encoding a Clostridial neurotoxin.

10. A vector comprising the nucleic acid sequence of claim 7 or 8, or the nucleic acid obtainable by the method of claim 9.

11. A eukaryotic host cell comprising the nucleic acid sequence of claim 7 or 8, or the vector of claim 10.

12. A method for producing the recombinant Clostridial neurotoxin of any one of claims 1 to 9, comprising the step of expressing the nucleic acid of claim 7 or 8, the nucleic obtainable by the method of claim 9, or the vector of claim 10 in a eukaryotic host cell, or cultivating the eukaryotic host cell of claim 11 under conditions that result in the expression of the nucleic acid sequence.

13. A method for detecting, isolating or purifying the recombinant Clostridial neurotoxin of any one of claims 1 to 6, comprising the step of using an antibody with specificity for a cleaved cis-acting hydrolase element sequence.

14. A pharmaceutical composition comprising the recombinant Clostridial neurotoxin of any one of claims 1 to 6.

15. The pharmaceutical composition of claim 14 for the treatment of a disease or condition taken from the list of: cervical dystonia (spasmodic torticollis), blepharospasm, severe primary axillary hyperhidrosis, achalasia, chronic focal painful neuropathies, migraine and other headache disorders, and cosmetic or aesthetic applications.

## Amended claims

### Amended claims in accordance with Rule 137(2) EPC.

**1.** A recombinant Clostridial neurotoxin comprising: (i) a functionally active Clostridial neurotoxin light chain; and (ii) a functionally active Clostridial neurotoxin heavy chain, **characterized in that** said light chain comprises a cleaved cis-acting hydrolase element at the C-terminus of said light chain, and said heavy chain comprises a proline residue located at the N-terminus of said heavy chain.

**2.** The recombinant Clostridial neurotoxin of claim 1, which is a functional homologue of a Clostridium botulinum neurotoxin taken from the list of: Clostridium botulinum neurotoxin serotype A, B, C, D, E, F, and G.

**3.** The recombinant Clostridial neurotoxin of claim 1 or 2, wherein said light chain consists of (a) amino acid residues 2 to 438 of Clostridium botulinum neurotoxin serotype A; or of (b) a functionally active variant of the light chain defined in (a), fused to a cleaved cis-acting hydrolase element at the C-terminus of said light chain.

**4.** The recombinant Clostridial neurotoxin of claim 1 or 2, wherein said heavy chain consists of an N-terminal proline linked to (a) amino acid residues 449 to 1296 of Clostridium botulinum neurotoxin serotype A; or (b) a functionally active variant of the heavy chain defined in (a), fused to a cleaved cis-acting hydrolase element at the C-terminus of said light chain.

**5.** The recombinant Clostridial neurotoxin of any one of claim 1 to 4, wherein said light chain has the amino acid sequence as found in SEQ ID NO: 1, and wherein said heavy chain has the amino acid sequence as found in SEQ ID NO: 2.

**6.** The recombinant Clostridial neurotoxin of any one of claims 1 to 5, wherein said cleaved cis-acting hydrolase element is comprised in a cis-acting hydrolase element from (i) a virus taken from the list of: EMC-B, EMC-D, EMC-PV21, TME-GD7, TME-DA, DME-BEAN, Theiler's-Like Virus, Ljungan virus (174F), Ljungan virus (145SL), Ljungan virus (87-012), Ljungan virus (M1146), FMD-A10, FMD-A12, FMD-C1, FMD-01G, FMD-O1K, FMD-O (Taiwan), FMD-O/SK, FMD-SAT3, FMD-SAT2, ERAV, ERBV, ERV-3, PTV-1, PTV-2, PTV-3, PTV-4, PTV-5, PTV-6, PTV-7, PTV-8, PTV-9, PTV-10, PTV-11, CrPV, DCV, ABPV, ABPV isolate Poland 1, ABPV isolate Hungary 1, IFV, TaV, EEV, KBV, PnPV (a), PnPV (b), Ectropis oblique picorna-like virus (a), Ectropis oblique picorna-like virus (b), Providence virus (a), Providence virus (b), Providence virus (c), Bovine Rotavirus, Porcine Rotavirus, Human Rotavirus, Bombyx mori, Lymantria dispar, Dendrolimus punctatus; (ii) a Trypansoma spp. Repeated Sequence taken from the list of: T. brucei TSR1, T. brucei (CAB95325.1), T. brucei (CAB95342.1), T. brucei (CAB95559.1), T. cruzi AP Endonuclease, or (iii) a cis-acting hydrolase element that is a functional variant or a cis-acting hydrolase element listed in (i) or (ii).

**7.** A nucleic acid sequence encoding the recombinant Clostridial neurotoxin of any one of claims 1 to 6 comprising (i) a nucleic acid sequence encoding a functionally active Clostridial neurotoxin light chain, (ii) a nucleic acid sequence encoding a cis-acting hydrolase element, and (iii) a nucleic acid sequence encoding a functionally active Clostridial neurotoxin heavy chain.

**8.** The nucleic acid sequence of claim 7, wherein said nucleic acid has the sequence as found in SEQ ID NO: 5.

**9.** A method for obtaining the nucleic acid sequence of claim 7 or 8, comprising the step of inserting of a nucleic acid sequence encoding a cis-acting hydrolase element into a nucleic acid sequence encoding a Clostridial neurotoxin.

**10.** A vector comprising the nucleic acid sequence of claim 7 or 8, or the nucleic acid sequence obtainable by the method of claim 9.

**11.** A eukaryotic host cell comprising the nucleic acid sequence of claim 7 or 8, the nucleic acid sequence obtainable by the method of claim 9, or the vector of claim 10.

**12.** A method for producing the recombinant Clostridial neurotoxin of any one of claims 1 to 6, comprising the step of expressing the nucleic acid of claim 7 or 8, the nucleic obtainable by the method of claim 9, or the vector of claim 10 in a eukaryotic host cell, or cultivating the eukaryotic host cell of claim 11 under conditions that result in the expression of the nucleic acid sequence.

**13.** A method for detecting, isolating or purifying the recombinant Clostridial neurotoxin of any one of claims 1 to 6, comprising the step of using an antibody with specificity for a cleaved cis-acting hydrolase element sequence.

**14.** A pharmaceutical composition comprising the recombinant Clostridial neurotoxin of any one of claims 1 to 6.

**15.** The pharmaceutical composition of claim 14 for the treatment of a disease or condition taken from the list of: cervical dystonia (spasmodic torticollis), ble-pharospasm, severe primary axillary hyperhidrosis, achalasia, chronic focal painful neuropathies, migraine and other headache disorders, and cosmetic or aesthetic applications.
